# EUROPEAN PATENT APPLICATION

(11) **EP 4 600 207 A1**
(43) Date of publication of application: **13.08.2025**
(21) Application number: 23882739.8
(22) Date of filing: 27.10.2023
(51) Int. Cl.: C01B 25/40, A61K 33/42, A61P 1/04, A61P 29/00

(54) **ZINC POLYPHOSPHATE AND METHOD FOR PRODUCING SAME**

(30) Priority: 27.10.2022 JP 2022172210
(71) Applicant: Kamui Pharma, Inc., Asahikawa-shi, Hokkaido 078-8802 (JP); National University Corporation Asahikawa Medical University, Asahikawa, Hokkaido 078-8510 (JP); KNC Laboratories Co., Ltd., Kobe-shi, Hyogo 650-0047 (JP)
(72) Inventor: FUJIYA, Mikihiro, Asahikawa-shi, Hokkaido 078-8510 (JP); KONISHI, Hiroaki, Asahikawa-shi, Hokkaido 078-8510 (JP); OGAWA, Naoki, Asahikawa-shi, Hokkaido 078-8510 (JP); IKEDA, Kohei, Kobe-shi, Hyogo 650-0047 (JP); ITO, Saori, Kobe-shi, Hyogo 650-0047 (JP); MIYAHARA, Junichi, Kobe-shi, Hyogo 650-0047 (JP)
(74) Representative: J A Kemp LLP
(86) International application number: PCT/JP2023/038803
(87) International publication number: WO 2024/090537

(57) **Abstract**

The present invention relates to a novel zinc polyphosphate, use thereof, and a production method therefor. The present invention relates more specifically to a zinc polyphosphate characterized by showing a halo peak at 2θ = 4 to 7° in X-ray diffraction, a pharmaceutical composition containing the zinc polyphosphate as an active ingredient, and a method for producing a zinc polyphosphate, the method comprising the steps of: (1) adding a zinc ion to a polyphosphate solution under an alkaline condition at preferably pH 8 to 11; and (2) recovering a solid obtained in the step (1) as a zinc polyphosphate.

## Description

### Technical Field

### Related application:

The present specification encompasses the contents described in the specification of Japanese Patent Application No. 2022-172200 (filed on October 27, 2022), which is the basis for the priority of the present application.

### Technical Field:

The present invention relates to a novel zinc polyphosphate and a production method therefor.

### Background Art

Although anti-inflammatory drugs are used as standard treatments for inflammatory bowel disease (IBD) such as ulcerative colitis (UC) and Crohn's disease (CD), there are no drugs that directly induce mucosal healing. While conventional anti-inflammatory drugs have been used for symptomatic treatment of IBD, the true goal of treatment of IBD is nowadays said to be "mucosal healing". Probiotics such as lactic acid bacteria are known for their high safety and certain intestinal regulation effects based on many years of dietary experience, but the mechanism of action of probiotics on the intestinal condition is still unclear in many respects. In order to elucidate this, bioactive molecules produced by probiotics are being identified and analyzed.

The present inventors have identified a long-chain polyphosphate derived from malt lactic acid bacteria as a molecule that enhances the intestinal barrier function. Then, it has been demonstrated that the long-chain polyphosphate improves decreased intestinal barrier function and intestinal injury caused by DSS treatment (see Patent Literature 1), and that it causes mucosal healing in patients with refractory ulcerative colitis (see Non Patent Literature 1). In addition, it has been reported that a Ca salt of polyphosphoric acid acts on the gastrointestinal mucosa injured in inflammatory bowel disease and exerts a specific platelet aggregation effect on the injured mucosa (Patent Literature 2).

Polyphosphate is known to have wound-healing and anti-inflammatory effects, and the development of wound dressing and dental materials using amorphous material or nanoparticles composed of calcium polyphosphate is expected (Patent Literature 3).

Polyphosphate is also known as a coagulant factor. When polyphosphate is released from platelets in blood, it activates factor XII protease in the blood, leading to a coagulation reaction. Various salts of natural polyphosphoric acid, such as Ca, Mg, Na, and K salts, have been postulated, and amorphous nanoparticles are also known to exist (Non Patent Literatures 2 and 3). Donovan *et al.* prepared nanoparticles from polyphosphate with different chain lengths and reported the size of polyphosphate and its effect on blood clotting (Non Patent Literature 4).

### Citation List

### Patent Literature

Patent Literature 1: WO 2011/125619
Patent Literature 2: WO 2021/141066
Patent Literature 3: WO 2016/079006

### Non Patent Literature

Non Patent Literature 1: Fujita et al., "Long-Chain Polyphosphate Is a Potential Agent for Inducing Mucosal Healing of the Colon in Ulcerative Colitis." Clin Pharmacol Ther. 2019 Sep.
Non Patent Literature 2: Feng et al., "Biogenic Polyphosphate Nanoparticles from a Marine Cyanobacterium Synechococcus sp. PCC 7002: Production, Characterization, and Anti-Inflammatory Properties In Vitro." Mar Drugs. 2018 Sep 10;16(9).
Non Patent Literature 3: Feng et al., "Biogenic Polyphosphate Nanoparticles from Synechococcus sp. PCC 7002 Exhibit Intestinal Protective Potential in Human Intestinal Epithelial Cells In Vitro and Murine Small Intestine Ex Vivo." J Agric Food Chem. 2018 Aug 1;66(30):8026-8035.
Non Patent Literature 4: Donovan et al., "Size-controlled synthesis of granular polyphosphate nanoparticles at physiologic salt concentrations for blood clotting." Biomacromolecules. 2014 Nov 10;15(11):3976-84

### Summary of Invention

### Technical Problem

The objective of the present invention is to provide a novel zinc polyphosphate, its use as a pharmaceutical agent, etc., and a production method therefor.

### Solution to Problem

The present inventors have found that a zinc polyphosphate obtained under certain conditions exhibits characteristic X-ray diffraction peaks and has excellent intestinal barrier enhancing effects.

The present invention is based on the above findings and, in the first aspect, provides the following [1] to [12].
[1] A zinc polyphosphate characterized by showing a halo peak at 2θ = 4 to 7° in X-ray diffraction.
[2] The zinc polyphosphate according to [1], wherein a zeta potential is - 20 mV or less.
[3] The zinc polyphosphate according to [1], wherein a zinc content is from 10 to 60%, preferably from 20 to 55%, and more preferably from 30 to 50%.
[4] The pharmaceutical composition according to [1], wherein an average chain length of a polyphosphate moiety of the zinc polyphosphate is 1.1 phosphate units or more and less than 100 phosphate units, preferably 1.1 phosphate units or more and less than 50 or less phosphate units, more preferably 1.2 to 30 phosphate units, and still more preferably 1.2 to 15 phosphate units.
[5] The zinc polyphosphate according to [1], wherein an average particle size of the zinc polyphosphate is from 1 to 1000 nm and more preferably from 20 to 500 nm.
[6] The zinc polyphosphate according to [1], wherein a half-life of intestinal barrier enhancing activity under a condition at 60°C or less and 75% humidity is 2 weeks or more, preferably 4 weeks or more, and still more preferably 6 months or more.
[7] A pharmaceutical composition comprising, as an active ingredient, the zinc polyphosphate according to any one of [1] to [6].
[8] A method for producing a zinc polyphosphate, comprising the steps of:
   (1) adding a zinc ion to a polyphosphate solution under an alkaline condition at preferably pH 8 to 11, more preferably pH 9 to 10.5, and still more preferably pH 9.5 to 10.0; and
   (2) recovering a solid obtained in step (1) as a zinc polyphosphate.
[9] A method for producing a zinc polyphosphate, comprising the steps of:
   (1) adding a zinc ion to a polyphosphate solution under an alkaline condition;
   (2) then adjusting the solution to an alkaline condition at preferably pH 8 to 11, more preferably pH 9 to 10.5, and still more preferably pH 9.5 to 10.0, and making a reaction for more than 2 hours, preferably 3 hours or more, and more preferably 4 hours or more; and
   (3) recovering a solid obtained in step (2) as a zinc polyphosphate.
[10] The method according to [8] or [9], wherein the resulting zinc polyphosphate shows a halo peak at 2θ = 4 to 7° in X-ray diffraction.
[11] The zinc polyphosphate according to [1], wherein the zinc polyphosphate is obtained by the method according to [8] or [9].
[12] The zinc polyphosphate according to [1] or [11], wherein additional signal peaks at 2θ = 30 to 36° and/or 56 to 62° are exhibited in the X-ray diffraction.

### Advantageous Effects of Invention

This invention provides a novel zinc polyphosphate with high intestinal barrier enhancing activity and a production method therefor.

### Brief Description of Drawings

[Figure 1] Figure 1 shows NMR charts of various zinc polyphosphates. A: Test 15-2, B: Test 15-6, C: Test 15-12, D: Test 40-1, E: Test 40-6-1, F: Test 40-6-2, G: chain-shortened Test 15-12.
[Figure 2] Figure 2 shows X-ray diffraction charts of various zinc polyphosphates. A: Test 15-2, B: Test 15-6, C: Test 15-12, D: Test 40-1, E: Test 40-6-1, F: Test 40-6-2, G: chain-shortened Test 15-12.
[Figure 3] Figure 3 shows the particle size distributions of zinc polyphosphates. A: Test 15-6, B: Test 40-6-1, C: Test 40-6-2.
[Figure 4] Figure 4 shows the intestinal barrier enhancing effect of zinc polyphosphate (amount of ³H-mannitol leaked out of the intestinal tract). From left, untreated (control), oxidative stress loaded (NH₂Cl), oxidative stress loaded + Test 15-12 treated (NH₂Cl + Test 15-12).
[Figure 5] Figure 5 shows a comparison of the intestinal barrier enhancing effect (amount of ³H-mannitol leaked out of the intestinal tract) between Test 40-6-1 and Test 40-6-2. From left, control, oxidative stress loaded/untreated (NH₂Cl), oxidative stress loaded + Test 40-6-1 treated (NH₂Cl + Test 40-6-1), oxidative stress loaded + Test 40-6-2 treated (NH₂Cl + Test 40-6-2).

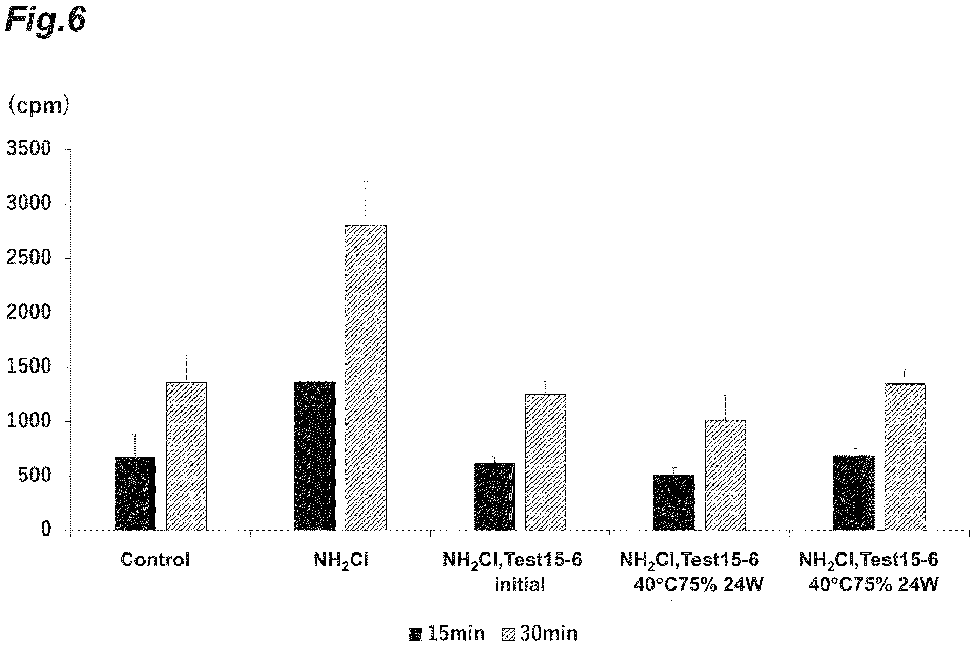

[Figure 6] Figure 6 shows the intestinal barrier enhancing effect (amount of ³H-mannitol leaked out of the intestinal tract) of zinc polyphosphate Test 15-6 after storage under conditions at 40°C and 75% RH or 60°C and 75% RH (RH: relative humidity). From left, control, oxidative stress loaded (NH₂Cl), oxidative stress loaded + Test 15-6 treated (NH₂Cl, Test 40-6-1), oxidative stress loaded + Test 15-6 treated, storage at 40°C and 75% RH for 24 weeks (NH₂Cl, Test 15-6, 40°C75% RH 24W), oxidative stress loaded + Test 15-6 treated, storage at 60°C and 75% RH for 24 weeks (NH₂Cl, Test 15-6, 60°C75% 24W).
[Figure 7] Figure 7 shows the intestinal barrier enhancement rate of zinc polyphosphate Test 15-12 or chain-shortened Test 15-12. From left, untreated (control), oxidative stress loaded (NH₂Cl), oxidative stress loaded + Test 15-12 treated (NH₂Cl + Test 15-12), oxidative stress loaded + chain-shortened Test 15-12 treated (NH₂Cl + Test 15-12).

### Description of Embodiments

### 1. Zinc polyphosphate

The present invention relates to a zinc polyphosphate characterized by showing a halo peak at 2θ = 4 to 7° in X-ray diffraction. The "zinc polyphosphate" means a zinc salt of polyphosphoric acid. In the above-mentioned "zinc polyphosphate," it is not necessary that all hydroxyl groups of phosphate (-[PO(OH)O]-) constituting the polyphosphate form zinc salts. The zinc polyphosphate may partly contain other metal salts, such as sodium polyphosphate or calcium polyphosphate, as long as the purpose of the present invention is not impaired. In addition, the zinc polyphosphate may also contain zinc hydroxide, zinc oxide, zinc phosphate and other zinc inorganic salts, calcium phosphate and other inorganic salts, calcium carbonate and other inorganic carbon compounds, as long as the purpose of the present invention is not impaired.

### (1) Polyphosphate

The "polyphosphate", which constitutes zinc polyphosphate, is a condensed phosphate compound composed of dehydrated and condensed phosphoric acid (H₃PO₄), and may be either linear, cyclic, or branched. Preferably, the polyphosphate is linear.

As used herein, the number (n) of "phosphate units" in the linear portion of polyphosphate is used to indicate the average chain length of the polyphosphate. For example, an average chain length of 10 phosphate units (n = 10) or more means that the number of repeats of phosphate units included in the linear portion of polyphosphate is 10 or more.

The "average chain length" of polyphosphate constituting the zinc polyphosphate of the present invention is not particularly limited, and the lower limit is, for example, 1.1 phosphate units or more, preferably 1.2 phosphate units or more (e.g., 1.3, 1.4, 1.5, 1.6, 1.7, 1.8, 1.9, 2.0 phosphate units or more). The upper limit is also not particularly limited, and the average chain length is, for instance, less than 100 phosphate units, preferably 50 phosphate units or less, more preferably 30 phosphate units or less, and still more preferably 15 phosphate units or less. For example, the "average chain length" of the polyphosphate of the present invention is in a range of 1.1 phosphate units or more and less than 100 phosphate units, preferably 1.1 to 50 phosphate units, more preferably 1.2 to 30 phosphate units, and still more preferably 1.2 to and 15 phosphate units. The zinc polyphosphate of the present invention, even when the chain is shortened, has a high intestinal barrier enhancing capacity and can effectively prevent or improve (cure or alleviate) inflammatory bowel disease.

The zinc polyphosphate of the present invention should be hardly soluble in water. Meanwhile, although the zinc polyphosphate of the present invention is preferably formed as nanoparticles, the form may be other than nanoparticles, such as a glass-like crushed material.

Polyphosphate may be chemically synthesized, synthesized *in vitro* using a biomolecule such as an enzyme, or synthesized using, for instance, polyphosphate-producing microorganisms. When linear polyphosphate is synthesized, it is preferable to synthesize it *in vitro* using a biomolecule such as an enzyme, or to synthesize it using microorganisms etc., because the linear polyphosphate can be produced with high efficiency.

Examples of a method of chemically synthesizing polyphosphate include a method in which a reaction solution containing sodium phosphate as a raw material is heated for dehydration and condensation. The heating temperature may be, for example, from 150 to 350°C.

Examples of the method of synthesizing polyphosphate *in vitro* using a biomolecule such as an enzyme include a method including: using, as the "biomolecule such as an enzyme", a polyphosphate kinase (PPK), which is an enzyme that synthesizes a polyphosphate; and synthesizing polyphosphate by an action of the PPK enzyme while using ATP as a raw material. It has been reported that many probiotics such as *Lactobacillus rhamnosus* GG strain and strains belonging to *Lactobacillus brevis* contain PPK, and the gene sequences of the corresponding enzymes have been published in DB.

PPK may be acceptable if polyphosphate can be synthesized using ATP as a substrate, may be obtained from a given microorganism strain expressing PPK, or may be a commercially available one that can be purchased. The PPK may be, for example, a PPK derived from *Propionibacterium freudenreichii* subsp. *shermanii.*

The enzymatic reaction by PPK is reversible, but when a large amount of ADP compared to ATP is present in the reaction solution, the polyphosphate degradation reaction becomes dominant so that the ADP/ATP ratio reaches equilibrium. Therefore, for efficient synthesis of polyphosphate, it is desirable to keep the ADP concentration in the reaction solution low, for example, by conjugating an ATP continuous regeneration reaction system with creatine kinase or pyruvate kinase. Other conditions such as the composition of the reaction solution, reaction temperature, and reaction time may be set, if appropriate, according to, for instance, the scale of synthesis to optimize the PPK activity.

As an example, the following shows the reaction conditions for the conjugation of a continuous ATP regeneration reaction system with pyruvate kinase during polyphosphate synthesis while using PPK derived from *Propionibacterium freudenreichii* subsp. *shermanii.* Mix 680 µL of 2 mol/L Tris-HCl pH 9.0, 0.1 g of phosphoenolpyruvate, 72 mg of adenosine 5'-trisphosphate disodium trihydrate, 160 µL of 1 mol/L phosphate buffer pH 6.0, 60 µL of 2 mol/L magnesium chloride, 1 mL of 2 mol/L acetic acid buffer pH 6.0, and 2.1 mL of purified water in a 10-mL sample tube, and heat the mixture at 40°C for 30 minutes. Add, after heating, 2.5 µL of 240 U/mL polyphosphate kinase, and further heat the mixture at 40°C for 5 minutes. Then, add 2.5 µL of 1690 U/mL pyruvate kinase, set the temperature to 40°C, where the reaction time may be set to 0.5 to 36 hours. The reaction time may be set, if appropriate, according to the molecular weight and yield of the target polyphosphate, and the reaction time is preferably about 20 hours to produce, for instance, polyphosphate with a high molecular weight in high yield.

Examples of the synthesis method using microorganisms include a method including culturing, under a suitable culture condition, a polyphosphate-producing microorganism, so that the microorganism can produce polyphosphate. Examples of the polyphosphate-producing microorganism include *Lactobacillus rhamnosus* GG strain, *Lactobacillus brevis* SBC8803 strain, strains belonging to *Lactobacillus,* strains belonging to *Bifidobacterium,* strains belonging to *Enterococcus,* strains belonging to *Lactococcus,* strains belonging to *Pediococcus,* strains belonging to *Leuconostoc,* strains belonging to *Streptococcus,* strains belonging to *Bacteroides,* strains belonging to *Eubacterium,* or strains belonging to *Clostridium.*

The polyphosphate may be synthesized by culturing the microorganism in an appropriate culture medium and under appropriate culture temperature conditions that can sustain the growth of the microorganism used. The synthesized polyphosphate may be recovered from the culture medium after culturing or by crushing the microorganism after culturing.

In the step of purifying the synthesized polyphosphate, it is possible to combine and use, if appropriate, purification methods generally used in the art, such as size exclusion chromatography, ion exchange chromatography, affinity chromatography, high performance liquid chromatography (HPLC), dialysis, salt precipitation, ammonium sulfate precipitation, sedimentation, and crystallization. The purification method used may be determined, if appropriate, according to, for instance, the method used in the polyphosphate synthesis process, the desired degree of purification, and the desired yield.

Preferably, the polyphosphate used in the preparation of the zinc polyphosphate of the present invention is linear polyphosphate obtained by an enzymatic synthesis process.

### (2) Zinc polyphosphate

The "zinc content" of the zinc polyphosphate of the present invention is preferably from 10 to 60%, more preferably from 20 to 55%, and still more preferably from 30 to 50%. The "zinc content" is particularly preferably from 32.5 to 42.5% for enzymatically synthesized long-chain polyphosphate as a raw material, and is particularly preferably from 40 to 50% for chemically synthesized short-chain polyphosphate as a raw material. Zinc polyphosphate in this range can achieve high intestinal barrier enhancing effect. Note that the "zinc content" refers to the percentage (%) by weight of the element zinc in the bulk zinc polyphosphate free of excipients and so on.

The zinc polyphosphate may contain some divalent metal salts of polyphosphoric acid such as calcium or magnesium salts, or monovalent metal salts such as sodium or potassium salts. In such cases, the content of a metal(s) other than zinc is in the range of 0 to 30%, preferably 0 to 20%, more preferably 0 to 10%, and still more preferably 0 to 7%. Note that the "content of a metal(s) other than zinc" refers to the percentage (%) by weight of the element metal(s) other than zinc in the bulk zinc polyphosphate free of excipients and so on.

The zeta potential of the zinc polyphosphate of the present invention is preferably -20 mV or less, and particularly preferably -30 mV or less. The present inventors have found that a change in the zeta potential of a polyphosphoric acid divalent metal salt correlates with a change in the intestinal barrier enhancement rate of the polyphosphoric acid divalent metal salt. Therefore, the intestinal barrier enhancing activity of the zinc polyphosphate can be predicted based on the change in the zeta potential of the zinc polyphosphate during storage.

In the present invention, the zinc polyphosphate is preferably hardly soluble in water, and should therefore be highly effective in efficiently reaching the intestinal mucosa, the site of action, and being taken up by epithelial cells through endocytosis. The water-insoluble zinc polyphosphate is also advantageous in terms of manufacturing cost because the bulk drug production step, such as the recovery, washing, and drying, is simple.

The zinc polyphosphate in the present invention is preferably "nanoparticles". The term "nanoparticles" means particles with a particle size on the order of nanometers. The nanoparticles are each often like a smooth sphere with a relatively uniform particle size, and have desirable properties in terms of formulation.

Specifically, the "average particle size" of the zinc polyphosphate of the present invention is preferably from 1 nm to 1000 nm and more preferably from 20 nm to 500 nm, and fine particles may aggregate to have a particle size in this range. The average particle size can be calculated from the particle size distribution according to known techniques.

The zinc polyphosphate according to the present invention is characterized by having a high "intestinal barrier enhancing effect" and also by being excellent in terms of storage stability. The intestine has an "intestinal barrier" function that protects the intestinal tract from the intake of pathogens and toxic substances. The term "intestinal barrier enhancing effect" or "intestinal barrier enhancing activity" refers to the action/activity of enhancing this intestinal barrier function. The "intestinal barrier enhancing effect" and "intestinal barrier enhancing activity" may be evaluated by comparing the intestinal permeability with that of the control, as demonstrated in the Examples described below. For example, in *ex vivo,* an intestine excised is filled with RPMI culture medium (Fujifilm Wako Pure Chemical Corporation) supplemented with a test substance and is cultured; next, ³H-mannitol (Perkin-Elmer) and a monochloramine solution (prepared by mixing distilled water, NH₄Cl (FUJIFILM Wako Pure Chemical Corporation), NaOCl (FUJIFILM Wako Pure Chemical Corporation), and RPMI culture medium (FUJIFILM Wako Pure Chemical Corporation)) is added to the culture medium and oxidative stress is thus applied; the amount of ³H-mannitol leaked out of the intestinal tract is measured; and the amount of ³H-mannitol in the case without the test substance is compared therewith to be able to evaluate the "intestinal barrier enhancing effect" and "intestinal barrier enhancing activity". The "intestinal barrier enhancing activity" may be evaluated by calculating the following intestinal barrier enhancement rate. Intestinal barrier enhancement rate (%) = {1 - (cpmsample - cpmcontrol) / (cpmNH2Cl - cpmcontrol)} × 100
cpmₛₐₘₚₗₑ: amount of ³H-mannitol under oxidative stress with polyphosphate addition
cpm_{NH2Cl}: amount of ³H-mannitol under oxidative stress (without polyphosphate addition)
cpm_{control}: normal amount of ³H-mannitol (without polyphosphate addition and without oxidative stress load)

For example, the half-life of the intestinal barrier enhancing activity (effect) of zinc polyphosphate of the present invention is 2 weeks or more, preferably 4 weeks or more, and still more preferably 6 months or more under the condition at 40°C and 75% humidity. Alternatively, the half-life of the intestinal barrier enhancing activity (effect) of zinc polyphosphate of the present invention is 2 weeks or more, preferably 4 weeks or more, and still more preferably 6 months or more under the condition at 60°C or less and 75% humidity.

The zinc polyphosphate of the present invention can maintain the above intestinal barrier enhancing activity even under an acidic condition. For example, the half-life of the intestinal barrier enhancing activity of the zinc polyphosphate of the present invention in an artificial gastric juice is 120 minutes or more and preferably 360 minutes or more.

### (3) X-ray diffraction chart

The zinc polyphosphate of the present invention is characterized by having a halo peak at 2θ = 4 to 7° in an X-ray diffraction chart. The halo peak means a broad peak that suggests properties different from typical amorphous properties. The zinc polyphosphate of the present invention should exhibit halo signal peaks at either of or preferably both 2θ = 30 to 36° and 56 to 62°.

### 2. Pharmaceutical composition containing zinc polyphosphate as active ingredient

The present invention also provides a pharmaceutical composition comprising zinc polyphosphate as an active ingredient. As described above, since zinc polyphosphate has intestinal barrier enhancing activity and storage stability, the pharmaceutical composition of the present invention is useful as a "pharmaceutical composition for prevention or treatment of inflammatory bowel disease" or "pharmaceutical composition for enhancing intestinal barrier". It is also promising as a pharmaceutical composition for the treatment of other diseases.

"Inflammatory Bowel Disease (IBD)" is a general term for chronic or remitting/relapsing inflammatory diseases of the intestinal tract, and generally refers to two diseases: Ulcerative Colitis (UC) and Crohn's Disease (CD). Ulcerative colitis is a diffuse, nonspecific inflammation of unknown cause, in which inflammation erosions and ulcers are formed on the mucosa of the large intestine, often with recurrence and remission. Crohn's disease is a general term for chronic inflammatory diseases of unknown cause, resulting in chronic inflammation and ulceration of the mucosa of the large and small intestine.

The zinc polyphosphate of the present invention also has a high intestinal barrier function-improving activity and thus effectively treats injured gastrointestinal mucosa and induces and maintains remission of inflammatory bowel disease. The present invention also provides such a "pharmaceutical composition for inducing/maintaining remission of inflammatory bowel disease".

The pharmaceutical composition of the present invention can inhibit expression of one or more inflammatory cytokines selected from intestinal inflammatory cytokines such as IL1β, TNF, IL6, and IL12B. The present invention also provides a "pharmaceutical composition for inhibiting expression of intestinal inflammatory cytokines", and the pharmaceutical composition comprising zinc polyphosphate of the present invention as an active ingredient.

The pharmaceutical composition of the present invention may contain a pharmacologically acceptable carrier and additives together with the active ingredient, zinc polyphosphate. Examples of such a carrier and additives include, but are not limited to, excipients, binders, lubricants, solvents, disintegrants, dissolution aids, suspending agents, emulsifiers, isotonic agents, stabilizers, preservatives, antioxidants, taste masking agents, coloring agents, buffers, and flow promoters, and other common carriers and additives may be used as needed.

Specifically, excipients include organic excipients, e.g., lactose, glucose, D-mannitol and other sugars, starches, crystalline cellulose and other celluloses and inorganic excipients, e.g., calcium carbonate, kaolin.

Examples of the binders include α-starch, gelatin, gum arabic, methylcellulose, carboxymethylcellulose, sodium carboxymethylcellulose, crystalline cellulose, D-mannitol, trehalose, hydroxypropyl cellulose, hydroxypropyl methylcellulose, polyvinylpyrrolidone, and polyvinyl alcohol.

Examples of the lubricants include stearic acid, fatty acid salts, e.g., stearate, talc, and silicates.

Examples of the solvents include purified water, physiological saline, and phosphate buffer.

Examples of the disintegrants include low-substituted hydroxypropyl cellulose, chemically modified cellulose and starches.

Examples of the dissolution aids include polyethylene glycol, propylene glycol, trehalose, benzyl benzoate, ethanol, sodium carbonate, sodium citrate, sodium salicylate, and sodium acetate.

Examples of the suspending agents or emulsifiers include sodium lauryl sulfate, gum arabic, gelatin, lecithin, glycerol monostearate, polyvinyl alcohol, polyvinyl pyrrolidone, celluloses such as sodium carboxymethyl cellulose, polysorbates, and polyoxyethylene hardened castor oil.

Examples of the isotonic agents include sodium chloride, potassium chloride, sugars, glycerin, and urea.

Examples of the stabilizers include polyethylene glycol, sodium dextran sulfate, other amino acids, and magnesium carbonate, which is also an acidity regulator.

Examples of the preservatives include paraoxybenzoates, chlorobutanol, benzyl alcohol, phenethyl alcohol, dehydroacetic acid, and sorbic acid.

Examples of the antioxidants include a water-soluble antioxidant, e.g., ascorbic acid, cysteine hydrochloride, sodium bisulfite, sodium metabisulfite, sodium sulfite, a fat-soluble antioxidant, e.g., ascorbyl palmitate, butylated hydroxyanisole (BHA), butylated hydroxytoluene (BHT), lecithin, propyl gallate, α-tocopherol, and a metal chelating agent, e.g., citric acid, ethylenediaminetetraacetic acid (EDTA), sorbitol, tartaric acid, phosphoric acid.

Examples of the taste and odor masking agents include sweeteners and flavoring agents, which are commonly used in the pharmaceutical field, and examples of the coloring agents include coloring agents commonly used in the pharmaceutical field.

The pharmaceutical composition of the present invention is a pharmaceutical preparation such as tablets (including sugar-coated tablets, film-coated tablets, sublingual tablets, orally disintegrating tablets), dispersions, granules, capsules (including soft capsules and microcapsules), liquids, troches, syrups, emulsions, suspensions, injectables (e.g., subcutaneous injectables, intramuscular injectables, intraperitoneal injectables), topical preparations (e.g., nasal preparations, transdermal preparations, ointment preparations), suppository (e.g., rectal suppository, vaginal suppository), foam preparations (intrarectal preparations), pellets, intranasal preparations, and transpulmonary preparations (inhalation preparations), and can be safely administered orally or parenterally (to an oral cavity, esophagus, stomach, small intestine, large intestine, rectum, etc.). The pharmaceutical composition of the present invention preferably involves oral administration, suppository administration, or rectum administration, and oral administration is particularly preferred.

The pharmaceutical composition of the present invention may be a controlled-release preparation such as a rapid-release preparation or a sustained-release preparation. In the case of oral preparation, coating may be applied for masking, enteric solubility, or persistence, if necessary. Examples of a coating base agent used for the coating include a sugar-coating base agent, a water-soluble film-coating base agent, an enteric-soluble film-coating base agent, and a sustained-release film-coating base agent.

The pharmaceutical composition of the present invention may be administered to humans or to non-human mammals. The dose and administration method are not particularly limited and may be determined, if appropriate, according to the condition, age, etc., of the individual receiving the drug.

The dose of the pharmaceutical composition of the present invention is determined, if appropriate, according to the purpose of use, route of administration, etc. For human administration, for example, the dose per day in terms of zinc polyphosphate may be selected from ranges of 0.01 mg/kg to 4 mg/kg, preferably 0.015 mg/kg to 2 mg/kg, and more preferably 0.03 mg/kg to 1 mg/kg. Alternatively, the pharmaceutical composition may be administered daily in one to several doses ranging from 0.6 to 240 mg/day, preferably 0.9 to 120 mg/day, and more preferably 1.8 to 60 mg/day per patient.

The zinc polyphosphate of the present invention is the main body of the intestinal barrier enhancing activity and includes zinc polyphosphate having excellent storage stability. Thus, the zinc polyphosphate of the present invention is expected to have higher efficacy and better storage stability than those of natural polyphosphates, which are miscellaneous mixtures, and unstable sodium polyphosphate.

The pharmaceutical composition of the present invention may be used in combination with other drugs as long as the purpose of the invention is not impaired. Examples of the pharmaceutical optionally used in combination with the pharmaceutical composition of the present invention include, but are not limited to, drugs commonly used for the treatment of inflammatory bowel disease such as ulcerative colitis and Crohn's disease, such as 5-aminosalicylic acid (5-ASA) preparations, e.g., mesalazine, salazosulfapyridine; steroids, e.g., prednisolone, methylprednisolone; anti-TNFα preparations, e.g., infliximab, adalimumab; anti-integrin antibody preparations, e.g., ENTYVIO; a JAK inhibitor Tofacitinib; thiopurine products, e.g., mercaptopurine, azathioprine; and immunosuppressive drugs, e.g., cyclosporine, tacrolimus. The timing of administration of the pharmaceutical composition of the present invention and the concomitant drug is not limited, and they may be administered at the same time or at different times.

Since the zinc polyphosphate of the present invention can be safely taken orally, it can be used as an ingredient in, for instance, foods for specified health uses, foods for special purposes, dietary supplements, health foods, functional foods, and foods for patients.

### 3. Method for producing zinc polyphosphate

The zinc polyphosphate of the present invention may be obtained by reacting polyphosphate with zinc chloride under an alkaline condition. For example, the pH of the polyphosphate solution is adjusted to 7.5 or more and preferably 8 to 11, and zinc chloride is then added and the resulting zinc polyphosphate is collected. Alternatively, zinc chloride is added to a polyphosphate solution under a non-alkaline condition, for example, at pH 4 to 7 or less, the pH is then adjusted to 7.5 or more and preferably 8 to 11, and the resulting zinc polyphosphate is collected.

For example, the zinc polyphosphate of the present invention may be produced by the following steps:
(1) adding a zinc ion to a polyphosphate solution under an alkaline condition at preferably pH 8 to 11, more preferably pH 9 to 10.5, and still more preferably pH 9.5 to 10.0; and
(2) recovering a solid obtained in step (1) as a zinc polyphosphate.

Alternatively, the zinc polyphosphate of the present invention may be produced by the following steps:
(1) adding a zinc ion to a polyphosphate solution under an alkaline condition;
(2) then adjusting the solution to an alkaline condition at preferably pH 8 to 11, more preferably pH 9 to 10.5, and still more preferably pH 9.5 to 10.0, and making a reaction (stirring or being allowed to stand) for more than 2 hours, preferably 3 hours or more, and more preferably 4 hours or more; and
(3) recovering a solid obtained in step (2) as a zinc polyphosphate.

The polyphosphate used may be obtained by the method described in "(1) Polyphosphate " in the Section 1. As mentioned above, the chain of the resulting polyphosphate is shortened more than that of the starting polyphosphate (e.g., sodium polyphosphate).

The zinc polyphosphate of the present invention has an intestinal barrier function enhancing effect and inhibits expression of intestinal inflammatory cytokines (one or more selected from IL1β, TNF, IL6, and IL12B), and may be administered to a subject in need thereof to treat or prevent, for instance, inflammatory bowel disease.

### Examples

Hereinafter, the present invention is further described in detail with reference to Examples. The present invention, however, is not limited to these Examples.

### Example 1: To prepare zinc polyphosphate (PPA-Zn)

### 1. Preparation of sodium polyphosphate (PPA-Na) (enzymatic process)

First, 680 µL of 2 mol/L Tris-HCl pH 9.0, 0.1 g of phosphoenolpyruvate, 72 mg of adenosine 5'-trisphosphate disodium trihydrate, 160 µL of 1 mol/L phosphate buffer pH 6.0, 60 µL of 2 mol/L magnesium chloride, 1 mL of 2 mol/L acetic acid buffer pH 6.0, and 2.1 mL of purified water were mixed in a 10-mL sample tube, and the mixture was heated at 40°C for 30 minutes. After heating, 2.5 µL of 240 U/mL polyphosphate kinase was added, and the mixture was further heated at 40°C for 5 minutes. Next, 2.5 µL of 1690 U/mL pyruvate kinase was added and allowed to react at 40°C for 20 hours. The reaction solution was admixed with 1.7 mL of 2 mol/L sodium chloride, and the mixture was stirred at 10°C for 10 minutes and allowed to stand. The supernatant was removed, 400 µL of purified water was added to the precipitate to dissolve it, and then 400 µL of 3 mol/L sodium chloride was added to cause a precipitate. The precipitate was further admixed with 400 µl of purified water for dissolution, and then 400 µL of 3 mol/L sodium chloride was added to cause a precipitate. After removing the supernatant, the precipitate was lyophilized to obtain 92 mg of long-chain sodium polyphosphate (PPA-Na) with a chain length of 600 mer or more.

### 2. To prepare various kinds of zinc polyphosphate

### 2.1 Preparation of zinc polyphosphate Test 15-12

To 1 g of sodium polyphosphate (PPA-Na) prepared in the Section 1., 50 g of water was added for dissolution. 1 mol/L aqueous sodium hydroxide solution was added, the pH was adjusted to 10.0, and 5 mL of 1 mol/L zinc chloride solution was added dropwise over 30 minutes. Aqueous sodium hydroxide solution was added as needed to maintain the pH between 9.8 and 10.1 during the dropping. After completion of the dropping, the mixture was stirred at room temperature for 4 hours, and then allowed to stand at a temperature below 10°C or less for 16 hours; and the precipitate was collected as a precipitate by centrifugation (8000 × g, 4°C, 10 minutes). The precipitate was further washed with aqueous ethanol (33 v/v% ethanol), and the precipitate was again collected by centrifugation and lyophilized to prepare 836 mg of zinc polyphosphate Test 15-12 as white powder.

### 2.2 Preparation of zinc polyphosphate Test 15-6

To 10 g of sodium polyphosphate (PPA-Na) prepared in the Section 1., 500 g of water was added for dissolution. 1 mol/L aqueous sodium hydroxide solution was added, the pH was adjusted to 10.0, and 50 mL of 1 mol/L zinc chloride solution was added dropwise over 30 minutes. Aqueous sodium hydroxide was added as needed to maintain the pH between 9.8 and 10.1 during the dropping. After completion of the dropping, the mixture was stirred at room temperature for 4 hours, and then allowed to stand at a temperature below 10°C or less for 16 hours; and the precipitate was collected as a precipitate by centrifugation (12000 × g, 4°C, 10 minutes). The precipitate was further washed with aqueous ethanol (33 v/v% ethanol), and the precipitate was again collected by centrifugation and lyophilized to prepare 7.8 g of zinc polyphosphate Test 15-6 as white powder.

### 2.3 Preparation of zinc polyphosphate Test 15-2

The same procedure as in the Section 2.1 was used to prepare 812 mg of zinc polyphosphate Test 15-2 from 1 g of sodium polyphosphate. The centrifugation conditions for precipitate collection, however, were at 15,000 × g and 4°C for 10 minutes.

### 2.4 Preparation of zinc polyphosphate Test 40-1

To 1 g of sodium polyphosphate (PPA-Na) prepared in the Section 1., 50 g of water was added for dissolution (pH 5.3). To this, 5 mL of 1 mol/L zinc chloride solution was added dropwise over 30 minutes. After completion of the dropping, 7.3 mL of 1 mol/L sodium hydroxide solution was added, the pH was adjusted to 10.0, and the mixture was stirred for another 4 hours. The mixture was allowed to stand at 10°C or less for 16 hours or more, and the precipitate was collected with a centrifuge. The precipitate collected was further washed with aqueous ethanol (33 v/v% ethanol), and the precipitate was again collected by centrifugation (15000 × g, 4°C, 10 minutes) and lyophilized to prepare 703 mg of zinc polyphosphate Test 40-1.

### 2.5 Preparation of zinc polyphosphate Test 40-6-1

To 1 g of sodium polyphosphate (PPA-Na) prepared in the Section 1., 50 g of water was added for dissolution (pH 5.4). To this, 5 mL of 1 mol/L zinc chloride solution was added dropwise over 30 minutes. After completion of the dropping, 7.3 mL of 1 mol/L sodium hydroxide solution was added, and the pH was adjusted to 9.9. The mixture was stirred at room temperature for 2 hours, and the supernatant and precipitate were each collected by centrifugation (4000 × g, 4°C, 10 minutes). The precipitate was washed with water and ethanol in that order and lyophilized to yield 403 mg of zinc polyphosphate Test 40-6-1.

### 2.6 Preparation of zinc polyphosphate Test 40-6-2

The supernatant collected above was allowed to stand at a temperature 10°C or less for 16 hours; and the resulting precipitate was collected as a precipitate by centrifugation (15000 × g, 4°C, 10 minutes). The precipitate was further washed with water, and the precipitate was again collected by centrifugation and lyophilized to prepare 48 mg of zinc polyphosphate Test 40-6-2.

### 3. To analyze zinc polyphosphate

### 3.1 To check degree of polymerization of zinc polyphosphate by ³¹P NMR measurement

About 10 mg of zinc polyphosphate was dissolved in about 0.7 mL of about 190 mmol/L sodium citrate buffer (D₂O) at pH 5.5, and the analytical sample was subjected to ³¹P NMR measurement.
(i) Equipment used: Bruker Biospin 400 MHz
(ii) Analysis and analytical conditions: the signal at -5 ppm was the terminal phosphate group, the signal at -15 to -25 ppm was the internal phosphate group, and the total integrated value of the phosphate groups when the integrated value of the terminal phosphate group was set to 2 as a reference was calculated. Since the zinc polyphosphate is prepared from linear PPA-Na in an aqueous solution, cyclic and branched polyphosphates should be rare, so that the calculated total integrated value of all the phosphate groups is considered to approximate the degree of polymerization.

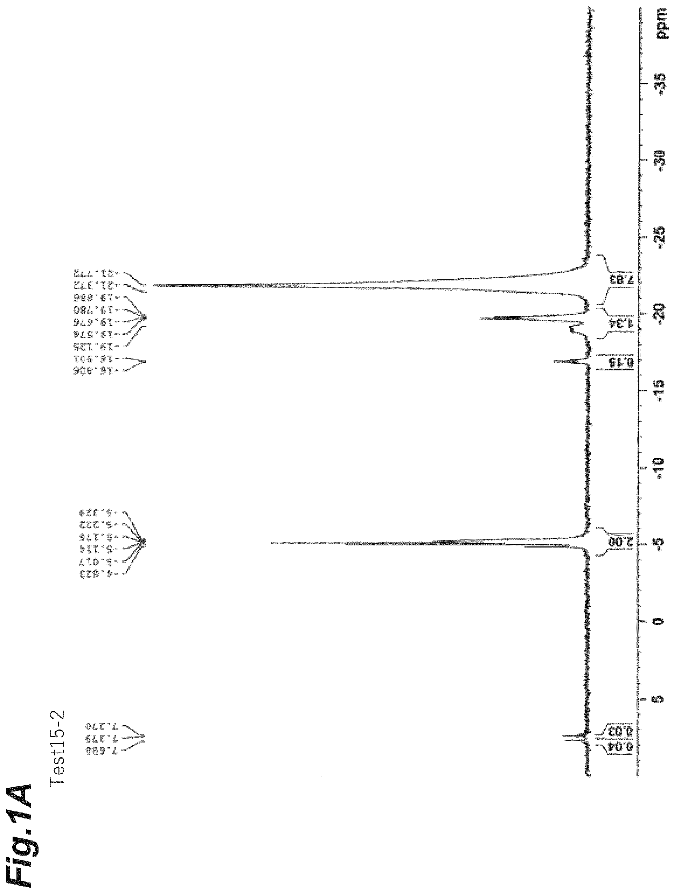

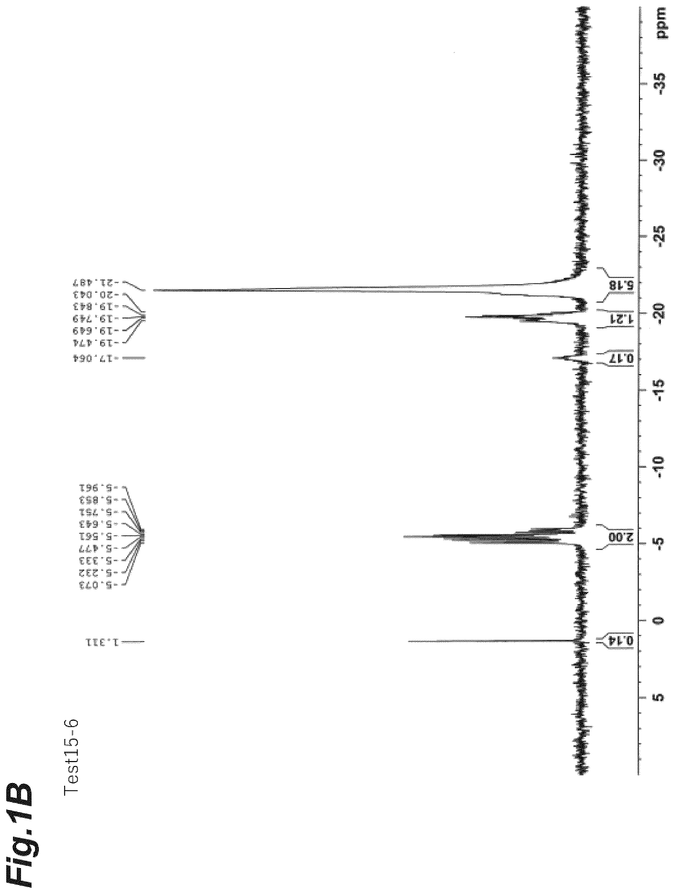

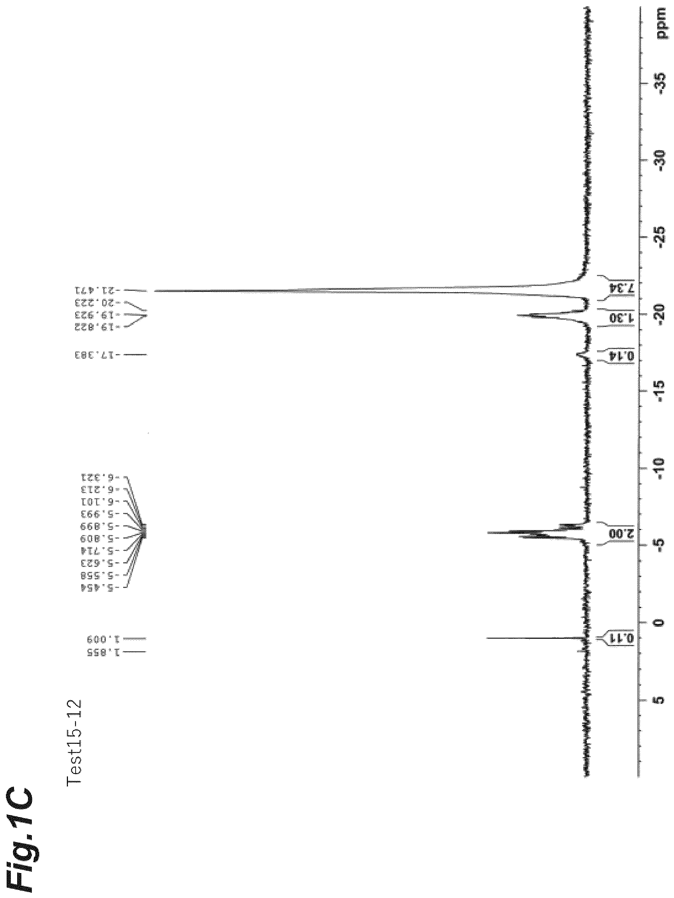

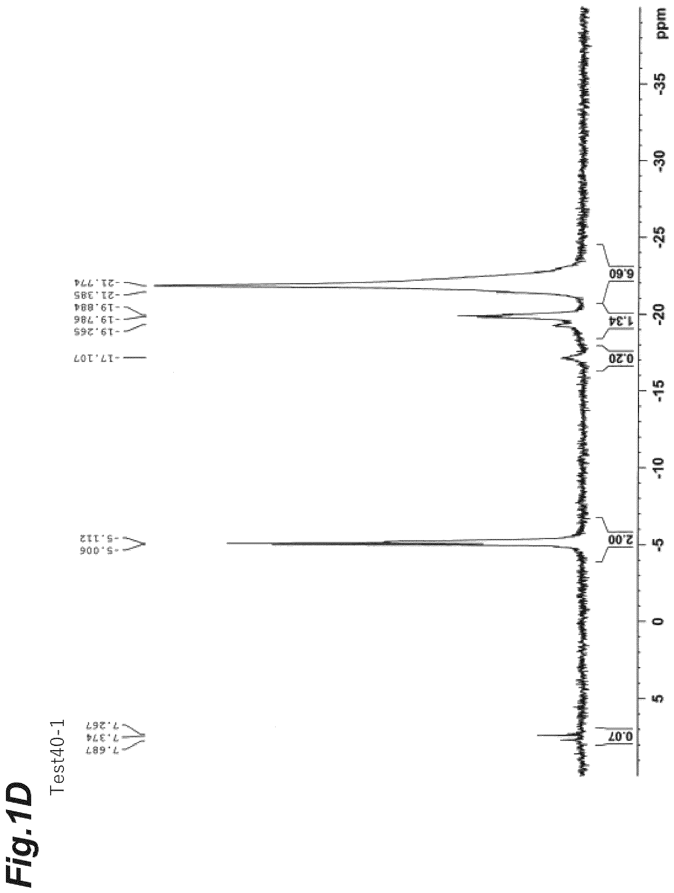

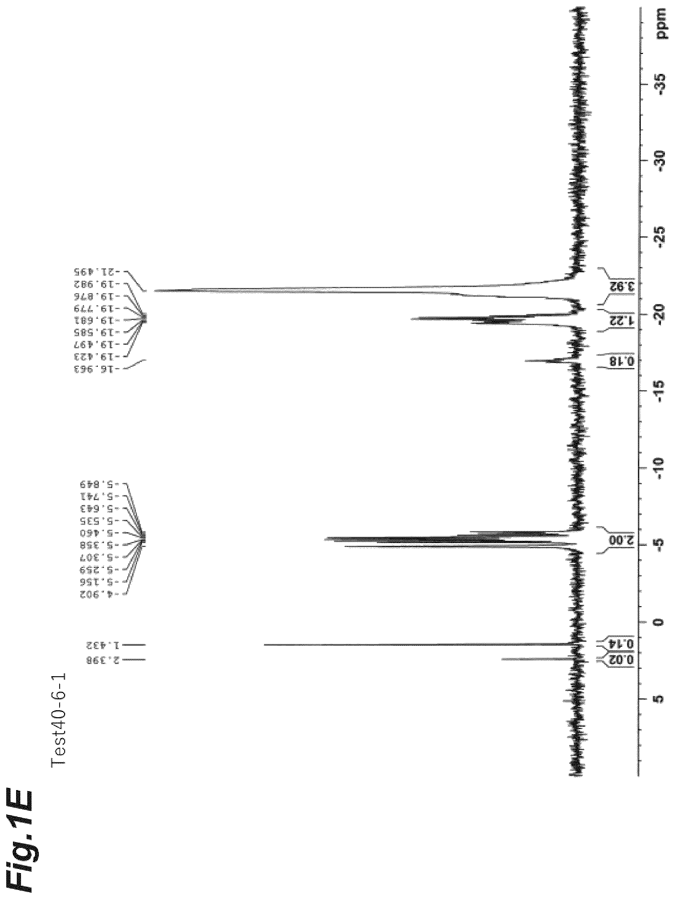

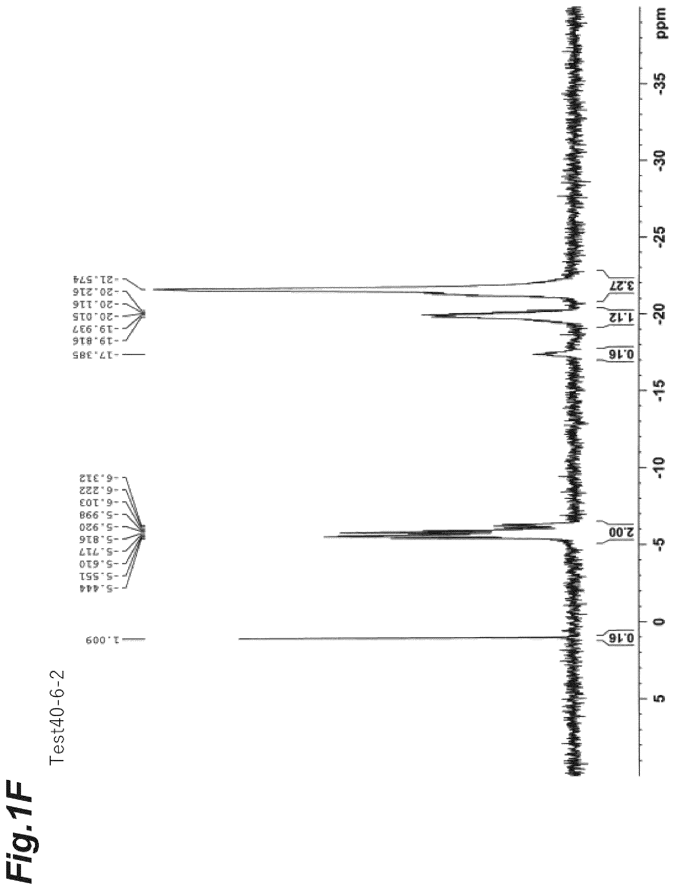

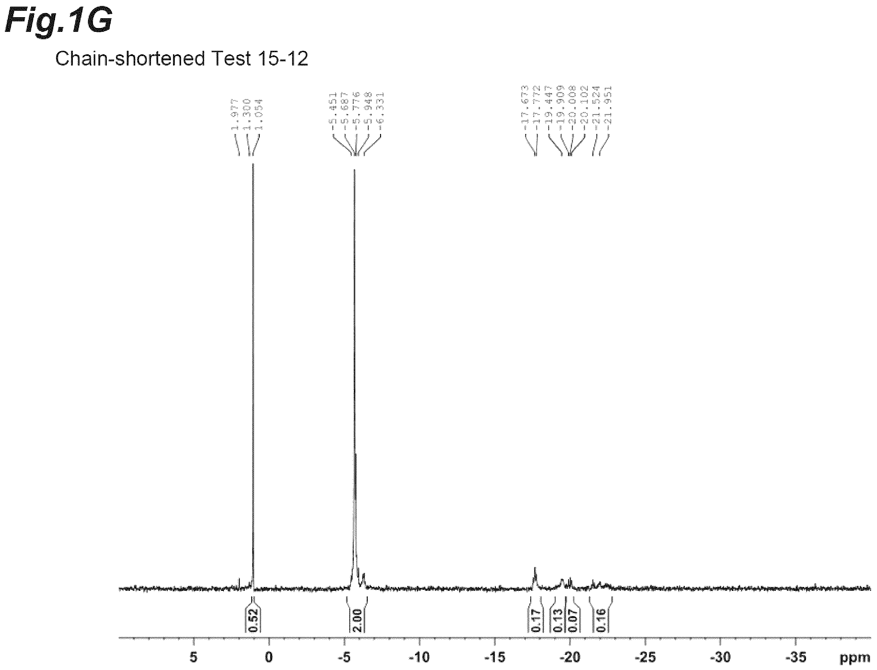

The NMR charts are shown in Figure 1 (A-F), and the calculated degree of polymerization (total integrated values of all the phosphate groups) are shown in Table 1. The degree of polymerization of each zinc polyphosphate was calculated to be in the relatively short chain range of 6.6-11.9, which was much shorter than that of the sodium polyphosphate (600 mer) used.

### 3.2 X-ray diffraction of zinc polyphosphate

10 to 50 mg of each zinc polyphosphate was pressed onto a glass plate, set in a crystal diffractometer, and measured.
(i) Equipment used: Rigaku MiniFlexII
(ii) Analysis conditions: cathode: Cu, tube voltage: 30 kV, tube current: 15 mA, monochromatization: Ni filter, sampling width: 0.020°, scanning speed: 10°C/min, wavelength: 1.541836 Å, measurement diffraction angle range (2θ): 2 to 60°, divergence slit: 1.25°, scattering slit: 8.0 mm, light receiving slit: open.

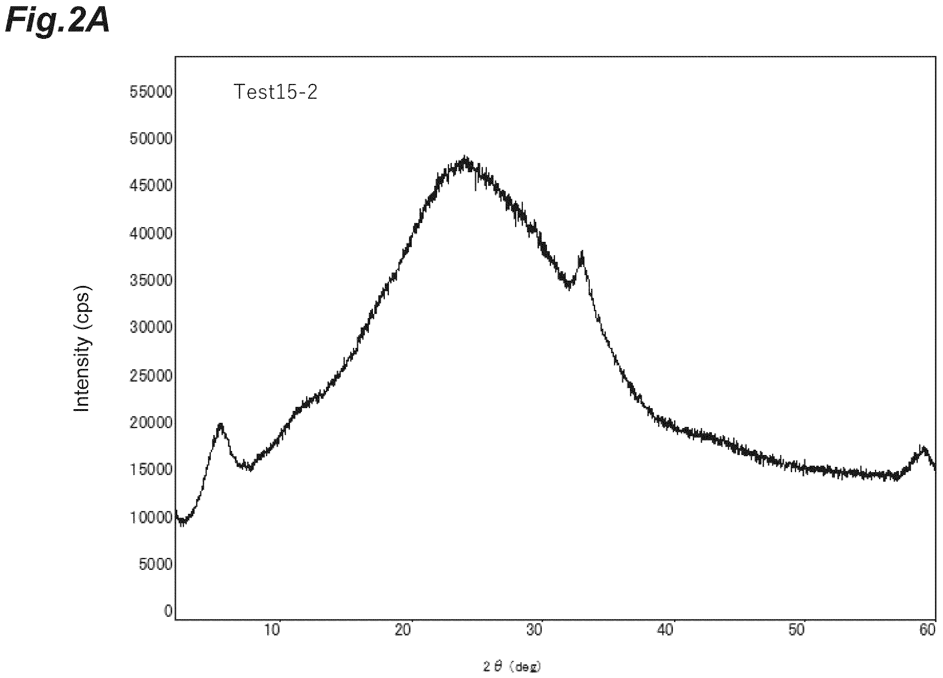

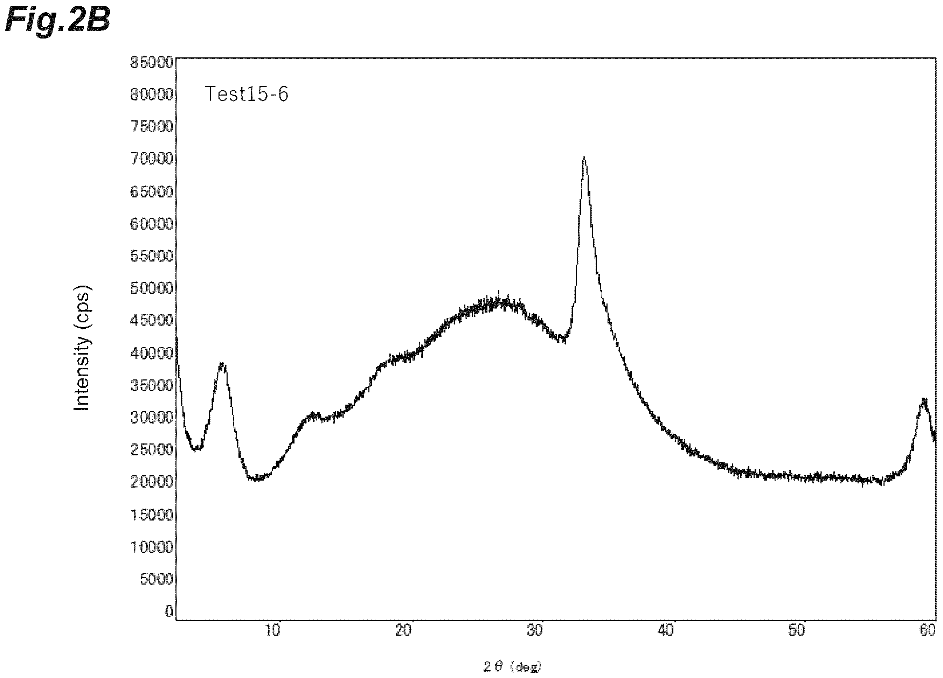

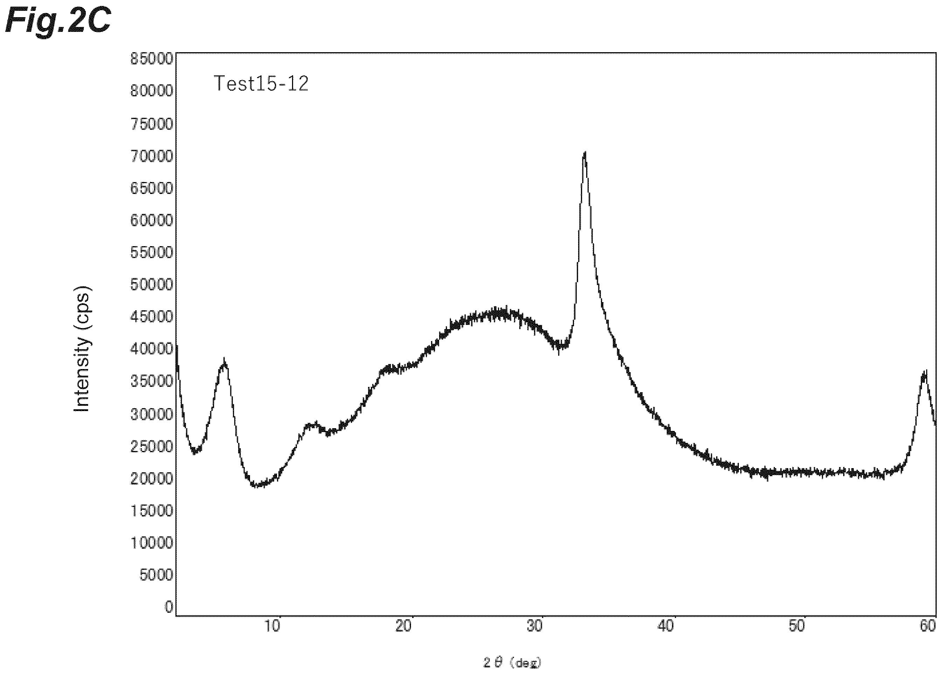

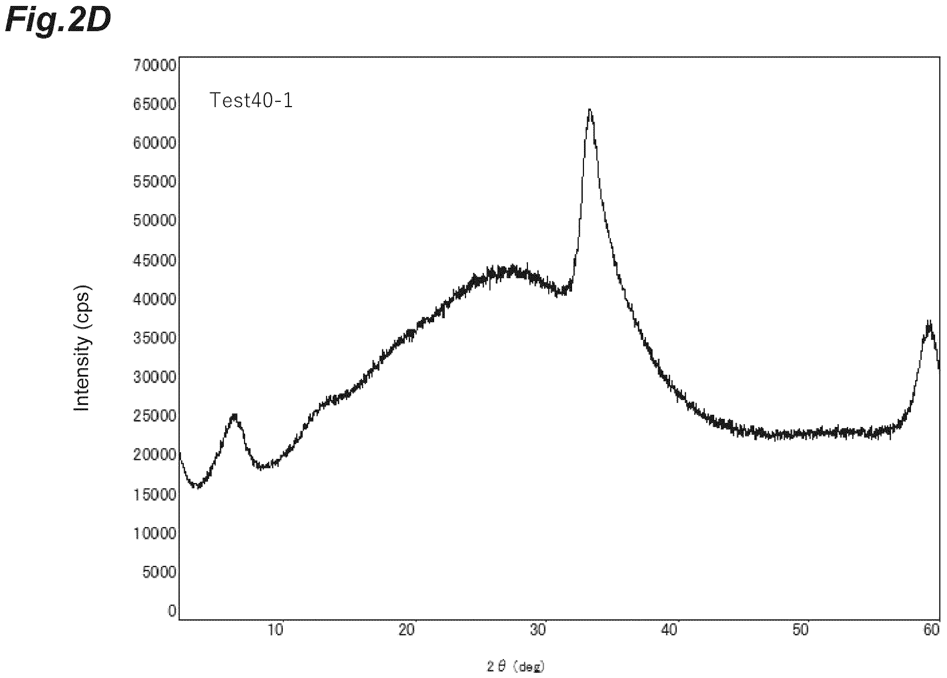

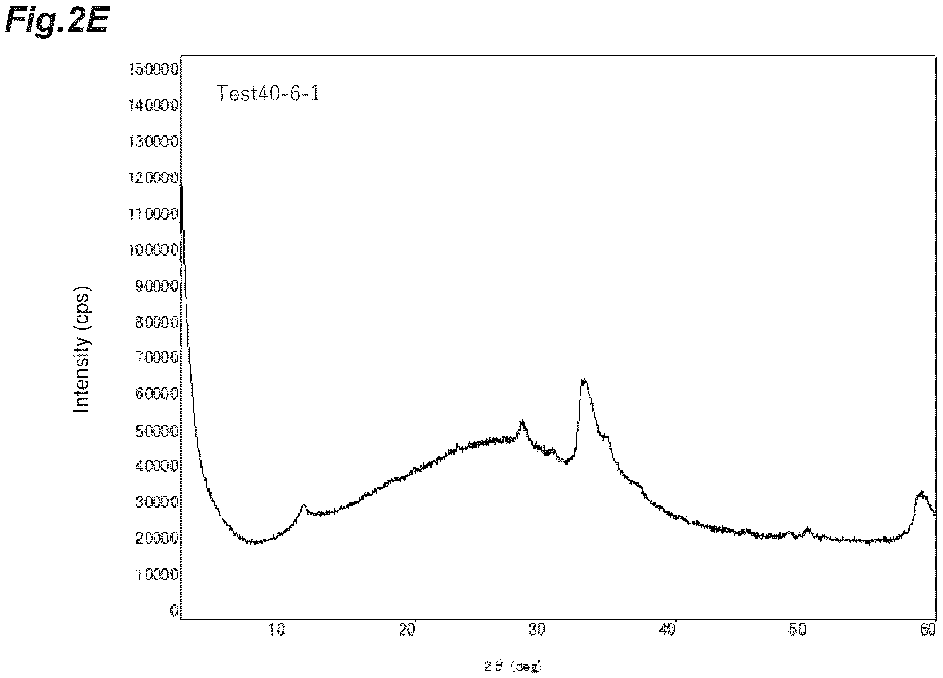

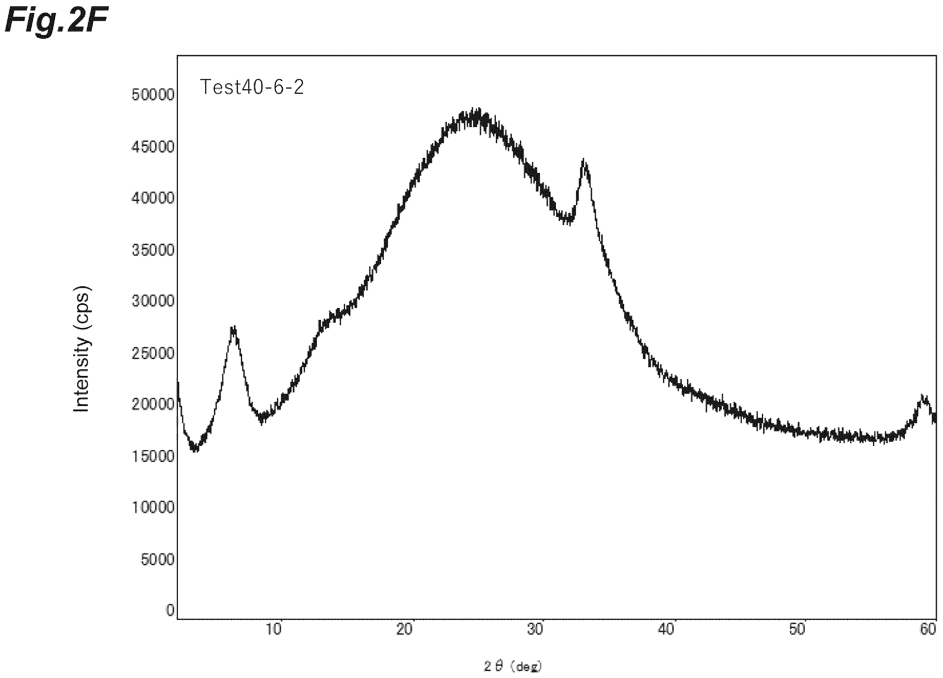

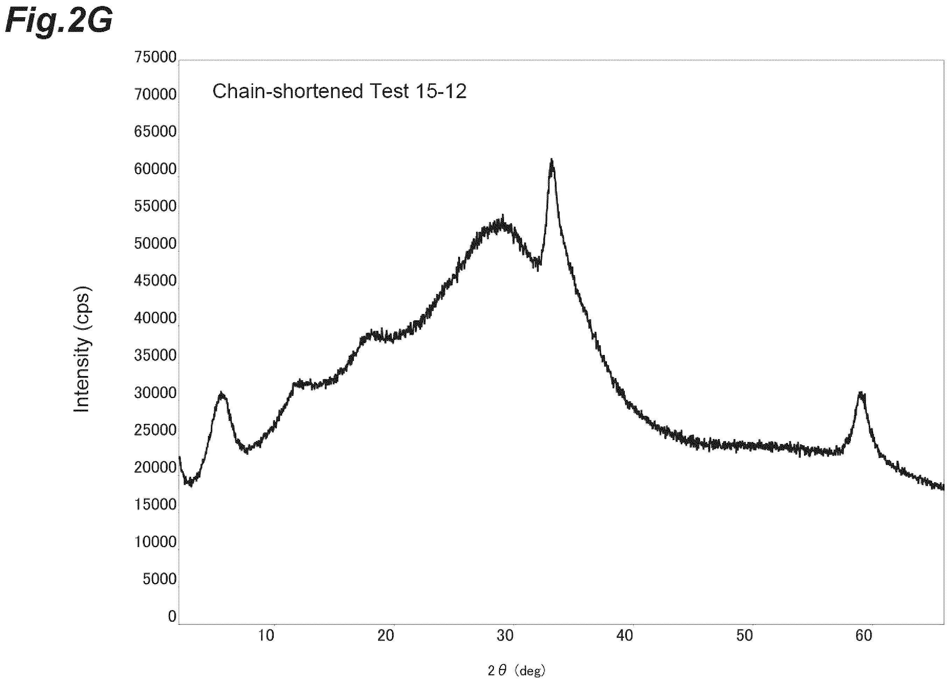

X-ray diffraction results are shown in Figure 2(A-F). In Test 15-2, Test 15-6, Test 15-12, Test 40-1, and Test4 0-6-2, halo signal peaks were observed at 2θ = 4 to 7°, 30 to 36°, and 56 to 62°. By contrast, in Test 40-6-1, halo signal peaks were observed at 2θ = 30 to 36° and 56 to 62°, but no peak at 2θ = 4 to 7°.

### 3.3 To measure pH, zeta potential, and particle size of zinc polyphosphate

5 mg of each zinc polyphosphate was suspended in 1 mL of purified water. The suspension was allowed to stand and the pH of the supernatant was measured. The suspension after pH measurement was dispersed by sonication and diluted to 2 mg/mL with purified water. The zeta potential and particle size were measured using this as an analytical sample.
(i) Equipment used: Malvern, ZEN3600
(ii) Analytical conditions: Dispersant: Water, Dispersant RI: 1.330, Viscosity (cp): 0.8872, Dispersant Dielectric Constant: 78.5, Temperature: 25°C

The results of measuring the pH and zeta potential, along with the degree of polymerization, are collectively provided in Table 1.

**[Table 1]**

| Zeta potential, pH, and NMR results | | | |
|---|---|---|---|
| | Zeta potential (Concentration 2 mg/mL) | pH (5 mg/mL Supernatant) | NMR: Degree of polymerization (mer) |
| Test 15-2 | -46.1 | 9.89 | 11.3 |
| Test 15-6 | -33.0 | 9.46 | 8.6 |
| Test 15-12 | -43.3 | 9.67 | 10.8 |
| Test 40-1 | -35.3 | 10.33 | 10.1 |
| Test 40-6-1 | -43.3 | 9.11 | 7.3 |
| Test 40-6-2 | -58.5 | 10.06 | 6.6 |

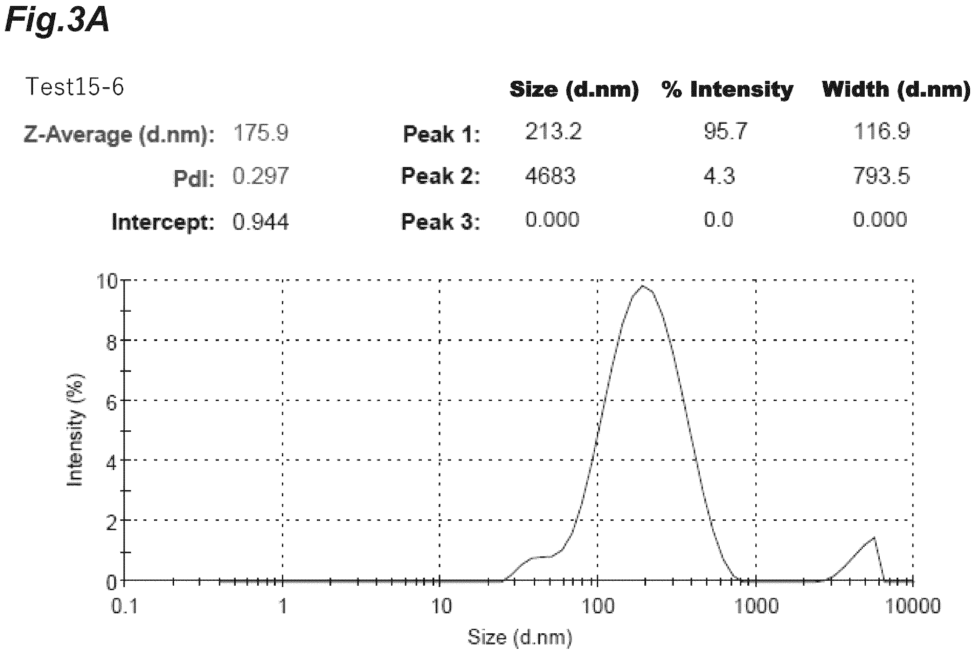

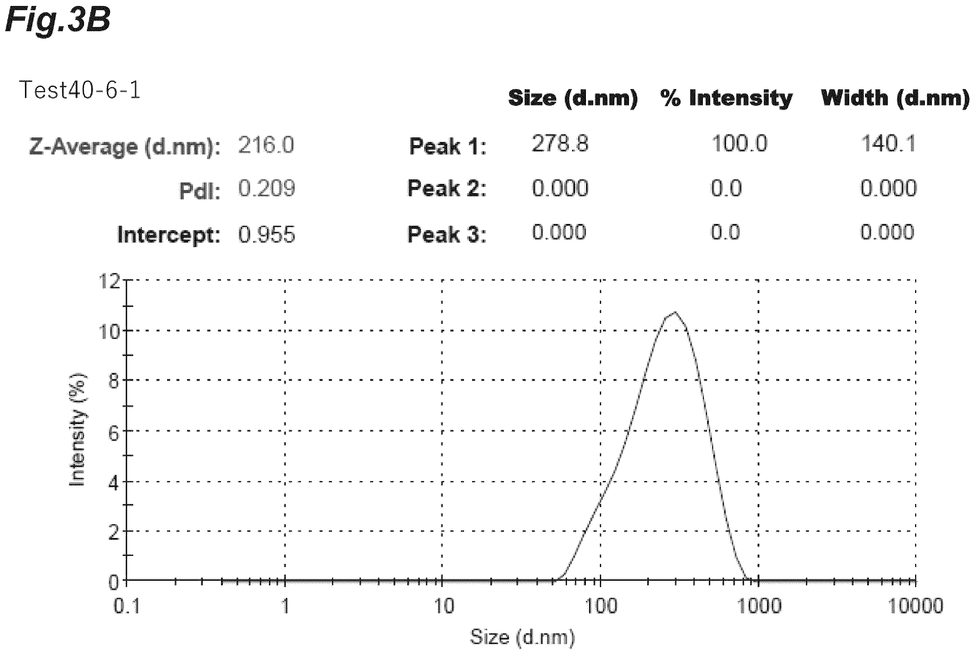

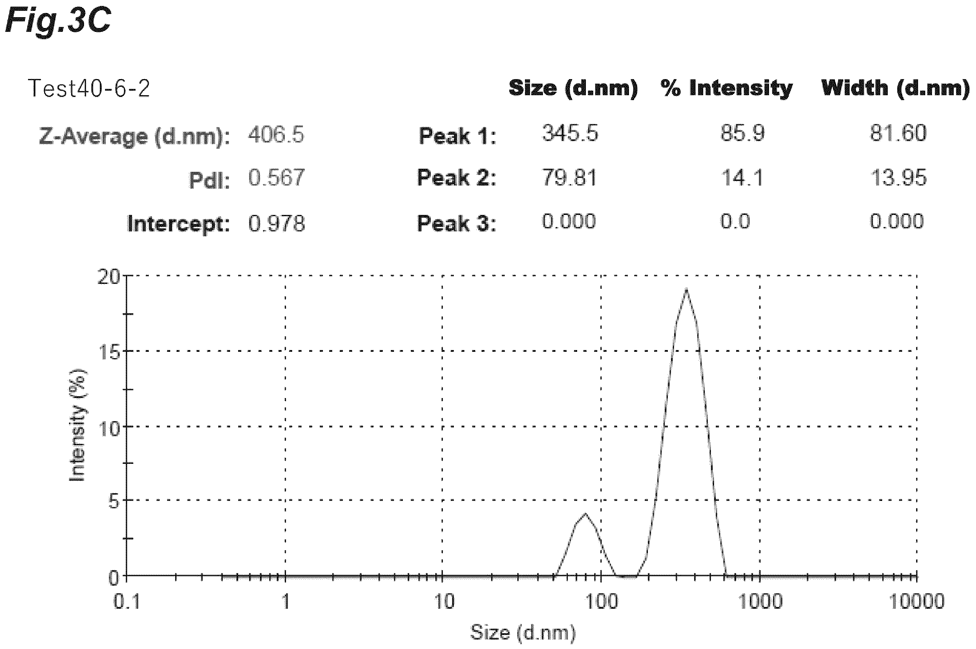

The results of measuring the particle size are shown in Figure 3. In many cases, the average particle size of zinc polyphosphate was detected around several hundred nm, but in some cases, a biphasic pattern with peaks and shoulders also detected in smaller sizes of several tens nm was observed, suggesting that fine particles may aggregate to form larger particles.

### Test Example 1: Effect of zinc polyphosphate Test 15-12 on enhancing intestinal barrier

### Ex vivo intestinal loop study

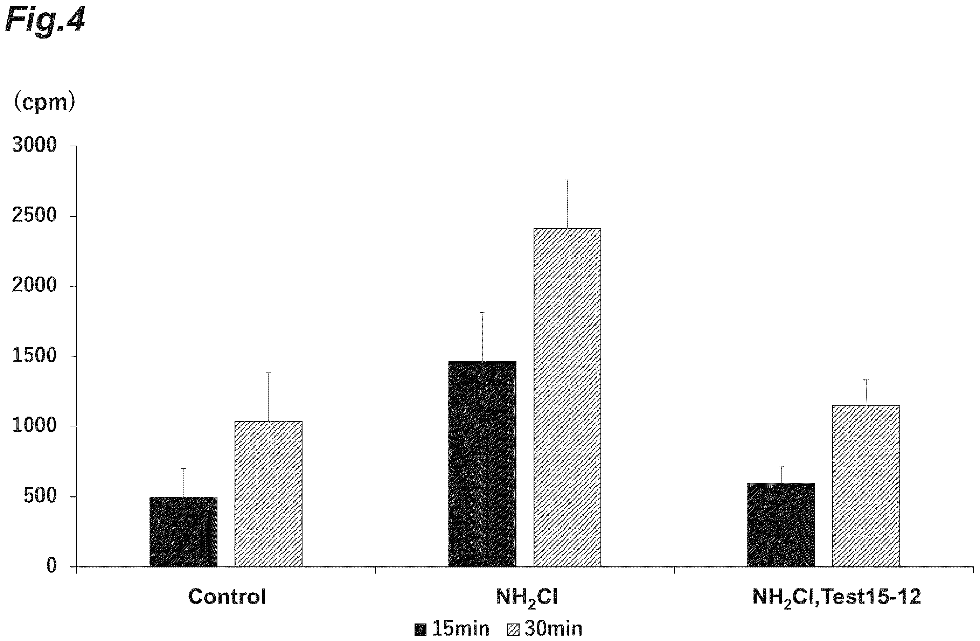

The intestinal tract from each C57BL/6 mouse was cut into four equal lengths, which were each ligated at the ends with silk sutures (thread number: 4-0; Alfresa Corporation), and the intestinal tract was filled with RPMI culture medium (FUJIFILM Wako Pure Chemical Corporation) containing 1 µg/mL of zinc polyphosphate Test 15-12. After incubation in RPMI culture medium at 37°C for 2 hours, one ligature was cut open, filled with a monochloramine solution containing 1 mCi/ml ³H-mannitol (Perkin-Elmer) at 1 µCi/mL, and ligated again, so that oxidative stress was loaded. Note that the monochloramine solution was prepared by mixing 920 µl of distilled water, 1000 µl of 40 mM NH₄Cl (FUJIFILM Wako Pure Chemical Corporation), 80 µl of 5.0% NaOCl (FUJIFILM Wako Pure Chemical Corporation), and 8000 µl of RPMI culture medium. The amount of ³H-mannitol leaked out of the intestinal tract 15 and 30 minutes after filling was measured with a liquid scintillator (Perkin Elmer; Liquid Scintillation Analyzer Tri-Carb 4910TR). In the same way, the amount of ³H-mannitol during oxidative stress load without polyphosphate addition and the amount of ³H-mannitol under the condition without polyphosphate addition or oxidative stress load (control) were measured (Figure 4).

As shown in Figure 4, when the zinc polyphosphate Test 15-12 was added, the amount of 3H-mannitol leaked out of the intestinal tract under oxidative stress load was comparable to the control, indicating that the zinc polyphosphate Test 15-12 exhibited the same high intestinal barrier enhancing effect.

### Test Example 2: Effect of zinc polyphosphate Test 40-6-1 or Test 40-6-2 on enhancing intestinal barrier

### Ex vivo intestinal loop study

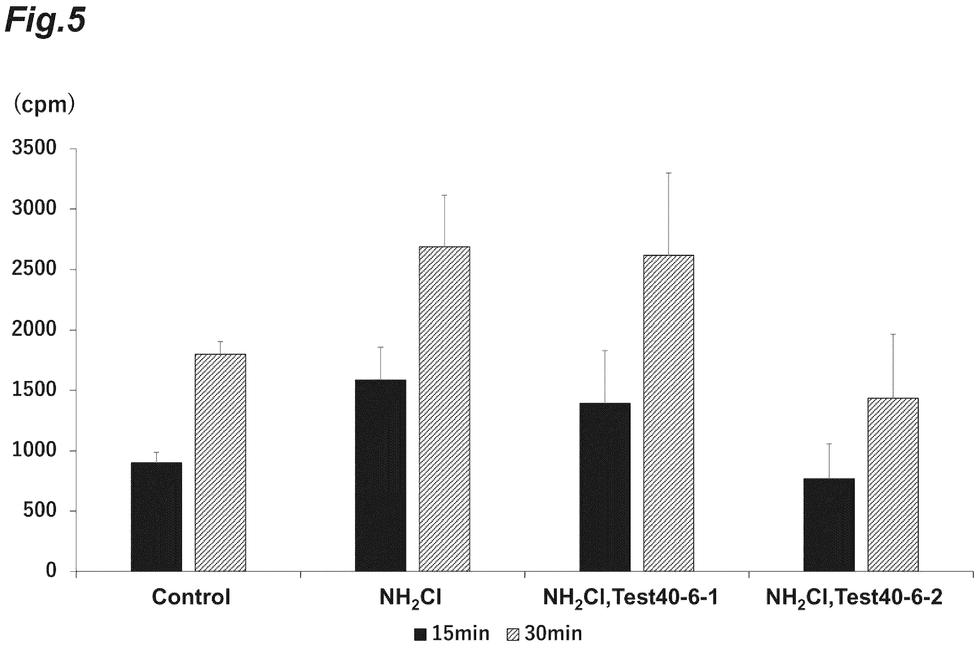

In accordance with the procedure described in Test Example 1, the intestinal barrier enhancing effect of zinc polyphosphate Test 40-6-1 or Test 40-6-2 was evaluated (Figure 5).

As shown in Figure 5, Test 40-6-2 exhibited a high intestinal barrier enhancing effect while the zinc polyphosphate Test 40-6-1 did not exert the intestinal barrier enhancing effect.

### Test Example 3: Stability of zinc polyphosphate

To compare (*ex vivo*) stability of Test 15-6 between condition of storage at 40°C + 75% humidity and condition of storage at 60°C + 75% humidity

### 1. Accelerated stability test

Zinc polyphosphate Test 15-6 was weighed in Eppendorf tubes at 20 mg each and stored in a small environmental tester (ESPEC CORP.; set to 40°C/75% RH) and a constant-temperature humidifier (Yamato Scientific Co., Ltd.; set to 60°C/75% RH) in the open state for 24 weeks.

### 2. Ex vivo intestinal loop study (40°C and 75% RH or 60°C and 75% RH, after storage)

In accordance with the procedure described in Test Example 1, the intestinal barrier enhancing effect of Test 15-6 after storage at 40°C and 75% RH or 60°C and 75% RH was evaluated (Figure 6).

As shown in Figure 6, zinc polyphosphate Test15-6 maintained its intestinal barrier enhancing effect for 24 weeks or more under the corresponding conditions.

### Test Example 4: Element analysis of each polyphosphate

20 mg of each polyphosphate (Test 15-2, Test 15-6, Test 15-12, Test 40-1, Test 40-6-1 or Test 40-6-2) was weighed on a precision electronic balance, and element analysis was performed at YAGAI-KAGAKU Co.,Ltd. (Sapporo). Moisture content was determined by the loss on drying method (drying at 105°C for 2 hours). A test liquid used was prepared by adding nitric acid and hydrogen peroxide to a sample, decomposing the sample by heat in a microwave sample pretreatment apparatus, and diluting the sample to 20 ml with distilled water. Sodium, calcium, or magnesium content was determined by a flame atomic absorption method using an atomic absorption spectrophotometer (Perkin Elmer, Analist 200), zinc content was determined by ICP mass spectrometry using an ICP mass analyzer (Agilent, Agilent 7500cx), and phosphorus content was determined by reduced molybdenum blue absorption spectrophotometry using a spectrophotometer (SHIMADZU CORPORATION, UV-1800) (Table 2).

**[Table 2]**

| Element analysis results | | | | | | |
|---|---|---|---|---|---|---|
| | Sodium (%) | Calcium (%) | Magnesium (%) | Zinc (%) | Phosphorus (%) | Moisture content (%) |
| PPA-Na | 17.80 | 0.01 | 1.89 | 0.001 | 30.6 | 5.0 |
| Test 15-2 | 6.27 | <0.005 | 0.737 | 36.9 | 14.9 | 7.9 |
| Test 15-6 | 4.56 | <0.005 | 0.837 | 39.6 | 15.4 | 9.6 |
| Test 15-12 | 5.78 | <0.005 | 0.832 | 34.6 | 15.2 | 3.9 |
| Test 40-1 | 4.87 | <0.005 | 0.835 | 37.3 | 13.8 | 1.5 |
| Test 40-6-1 | 2.85 | <0.005 | 0.770 | 40.4 | 12.2 | 3.8 |
| Test 40-6-2 | 2.55 | <0.005 | 0.677 | 42.6 | 13.5 | 9.4 |

### Test Example 5: Effect of chain-shortened Test 15-12 on enhancing intestinal barrier

Test 15-12 prepared in the Section 2.1 was stored in a closed container in a thermostatic bath at 40°C for 13 days to give a chain-shortened Test 15-12. When the degree of polymerization of the chain-shortened Test 15-12 was checked by ³¹P NMR measurement according to the procedure described in the Section 3.1, the degree of polymerization was shortened to 2.0 (Figure 1G). In accordance with the protocol described in the Section 3.2, when X-ray diffraction of zinc polyphosphate was measured, this chain-shortened Test 15-12 still had halo peaks at 2θ = 4 to 7°, etc., in the X-ray diffraction chart (Figure 2G). In accordance with the protocol described in the Section 3.3, when the pH and zeta potential of the chain-shortened Test 15-12 were measured, the zeta potential = -30.8 mV and pH = 8.3.

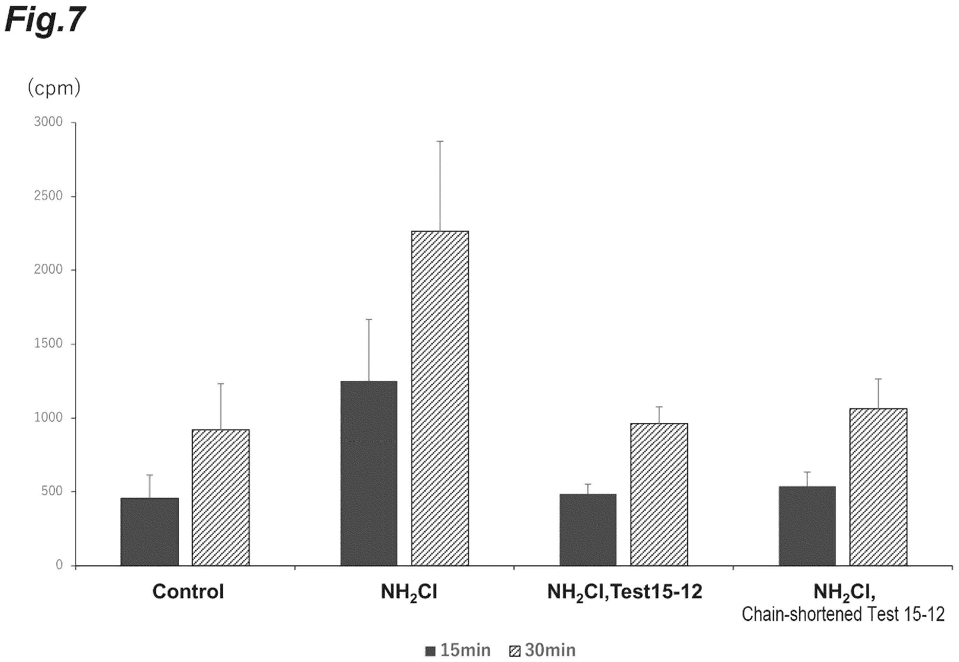

In accordance with the protocol described in Test Example 1, the intestinal barrier enhancing effects of zinc polyphosphate Test 15-12 and the chain-shortened Test 15-12 were determined. As shown in Figure 7, the chain-shortened Test 15-12 was added to suppress the amount of 3H-mannitol leaked out of the intestinal tract under oxidative stress load to the same level as the control, and the chain-shortened Test 15-12 thus exhibited a high intestinal barrier enhancing effect comparable to that of Test 15-12 (Figure 7). These results confirm that zinc polyphosphate has a high intestinal barrier enhancing effect even when the chain is shortened.

### Industrial Applicability

The zinc polyphosphate, which shows a halo peak at 2θ = 4 to 7° in X-ray diffraction, has an intestinal barrier enhancing effect and excellent storage stability, and is useful for the treatment and prevention of inflammatory bowel disease such as ulcerative colitis and Crohn's disease, and is thus promising as a pharmaceutical composition for the treatment of other diseases.

All the publications, patents, and patent applications cited herein are incorporated herein by reference in their entirety.

## Claims

1. A zinc polyphosphate **characterized by** showing a halo peak at 2θ = 4 to 7° in X-ray diffraction.

2. The zinc polyphosphate according to claim 1, wherein a zeta potential is -20 mV or less.

3. The zinc polyphosphate according to claim 1, wherein a zinc content is from 10 to 60%.

4. The zinc polyphosphate according to claim 1, wherein an average chain length of a polyphosphate moiety of the zinc polyphosphate is 1.1 phosphate units or more and less than 100 phosphate units.

5. The zinc polyphosphate according to claim 1, wherein an average particle size of the zinc polyphosphate is from 1 to 1000 nm.

6. The zinc polyphosphate according to claim 1, wherein a half-life of intestinal barrier enhancing activity under a condition at 60°C or less and 75% humidity is 2 weeks or more.

7. A pharmaceutical composition comprising, as an active ingredient, the zinc polyphosphate according to any one of claims 1 to 6.

8. A method for producing a zinc polyphosphate, comprising the steps of:
(1) adding a zinc ion to a polyphosphate solution under an alkaline condition at preferably pH 8 to 11; and
(2) recovering a solid obtained in step (1) as a zinc polyphosphate.

9. A method for producing a zinc polyphosphate, comprising the steps of:
(1) adding a zinc ion to a polyphosphate solution under an alkaline condition;
(2) then adjusting the solution to an alkaline condition at preferably pH 8 to 11, and making a reaction for more than 2 hours; and
(3) recovering a solid obtained in step (2) as a zinc polyphosphate.

10. The method according to claim 8 or 9, wherein the resulting zinc polyphosphate shows a halo peak at 2θ = 4 to 7° in X-ray diffraction.

11. The zinc polyphosphate according to claim 1, wherein the zinc polyphosphate is obtained by the method according to claim 8 or 9.
